# EUROPEAN PATENT APPLICATION

(11) **EP 1 902 683 A1**
(43) Date of publication of application: **26.03.2008**
(21) Application number: 07253699.8
(22) Date of filing: 18.09.2007
(51) Int. Cl.: A61B 18/14, A61B 18/00

(54) **A catheter assembly**

(30) Priority: 21.09.2006 US 846606 P
(71) Applicant: CATHRX LTD, Eveleigh, NSW 1430 (AU)
(72) Inventor: Anderson, Neil Lawrence, Roseville, New South Wales 2069 (AU)
(74) Representative: Nettleton, John Victor

(57) **Abstract**

A catheter sheath 10 includes an electrical lead 12 carrying a plurality of electrical conductors 16. A sleeve 20 is co-axially arranged about the electrical lead 12. At least one of a distal end of the lead 12 and a distal end of the sleeve 20 defines a plurality of displaceable petals 22, the petals 22 being displaceable between a first, inoperative position in which the petals 22 extend parallel to a longitudinal axis of the sleeve 20 and a second, operative position in which the petals 22 lie transversely to the longitudinal axis of the sleeve 20. At least one of the petals 20 carries at least one electrode 28, the, or each, electrode 28 being in electrical connection with a group of the conductors 16 of the lead 12.

## Description

### Cross-Reference to Related Applications

The present application claims priority from United States of America Provisional Patent Application No 60/846,606 filed on 21 September 2006, the contents of which are incorporated herein by reference.

### Field

This invention relates, generally, to the field of catheters and, more particularly, to a catheter assembly, to a catheter sheath for a catheter assembly and to a method of fabricating a catheter sheath.

### Background

In the field of heat treatment of tissue, it is desirable if the device heating the tissue is in contact only with the tissue being treated and not surrounding tissue or bodily fluids. This minimises the power required to heat the tissue and also minimises unnecessary damage to other tissue, structures or fluid.

In addition, it is often necessary to overcome tissue irregularities at a site in a patient's body being heat treated. An example where a site in a patient's body is subjected to heat treatment is in the treatment of heart arrhythmias where tissue is ablated in an effort to cure the arrhythmia. The tissue is ablated to create a lesion to block the electrical impulses causing the arrhythmia. To ensure that a lesion of adequate depth is formed, it is desirable that the ablating electrode make good contact with the tissue. Other examples of the use of heat treatment at a site in a patient's body include treatment of Parkinson's disease, tumour ablation, endometriosis and pain management.

Still further, in the treatment of arrhythmias, it may be necessary to ablate over a reasonably wide area in an attempt to cure the arrhythmia. It would be beneficial to be able to obtain such larger ablated areas with minimum manipulation of the catheter when in position at the site to be treated.

There is therefore a need for a catheter sheath and a catheter assembly which meets these needs. Such a catheter sheath and catheter assembly could also be useful in other applications, for example, pacing, sensing or defibrillation.

### Summary

According to a first aspect of the invention, there is provided a catheter sheath which includes
an electrical lead carrying a plurality of electrical conductors; and
a sleeve co-axially arranged about the electrical lead, at least one of a distal end of the lead and a distal end of the sleeve defining a plurality of displaceable petals, the petals being displaceable between a first, inoperative position in which the petals extend parallel to a longitudinal axis of the sleeve and a second, operative position in which the petals lie transversely to the longitudinal axis of the sleeve; and
at least one of the petals carrying at least one electrode, the, or each, electrode being in electrical connection with a group of the conductors of the lead.

The petals may be displaceable between their inoperative position and their operative position by relative movement between the electrical lead and the sleeve.

The electrical lead may be tubular defining a lumen and the electrical conductors may be embedded in a wall of the electrical lead.

The distal end of the sleeve may extend beyond the distal end of the lead, the distal end of the sleeve defining the petals. It will be appreciated that a part of each of the conductors protrudes beyond the distal end of the lead to be attached to a surface of its associated petal.

The sleeve may be of a plastics material of a first stiffness along its length and a more flexible material in a region of deflection in which region the sleeve is able to bend.

In an embodiment, the catheter may include a crown portion of a plastics material of a different stiffness to the flexible material of the region of deflection, the crown portion being attached to a distal end of the electrical lead and projecting distally of the distal end of the electrical lead, the crown portion defining a plurality of secondary leaves, each secondary leaf being in register with one of the petals of the sleeve. The crown portion may be arranged within the sleeve and the electrodes may be carried on inner surfaces of the secondary leaves of the crown portion, each secondary leaf being fast with its associated petal of the sleeve. By "inner surface" is meant that, when the petals are in their first, inoperative position, the inner surface of each petal is that surface facing an opposed petal.

In another embodiment, the sleeve may be of a plastics material of a first stiffness along its length, a more flexible material in a region of deflection in which region the sleeve bends and the sheath may include a crown portion of a plastics material of a different stiffness to the flexible material of the region of deflection attached, for example, by welding, to a distal end of the region of deflection, the crown portion defining the petals the sleeve.

In other embodiments, the sleeve may terminate short of the distal end of the lead with the lead defining the petals. The sleeve may be attached to an outer surface of each of the petals of the lead.

According to a second aspect of the invention, there is provided a method of fabricating a catheter sheath, the method including
providing an electrical lead carrying electrical conductors;
providing a sleeve and arranging the sleeve co-axially with respect to the electrical lead, at least one of the sleeve and the lead defining a plurality of discrete petals at its distal end;
forming at least one electrode on at least one of the petals; and
electrically connecting the, or each, electrode to a group of the conductors.

The method preferably includes rendering the group of conductors mechanically fast with its associated petal.

The electrical lead may be tubular defining a lumen with the conductors being embedded in a wall of the electrical lead, the method including removing material of a distal part of the wall of the electrical lead to expose distal regions of the conductors which form free parts of the conductors. The method may include arranging the parts of the conductors to extend distally from the distal end of the electrical lead. The method may include, after exposing the parts of the conductors, stripping insulation from the exposed parts of the conductors.

Further, the method may include arranging the sleeve about the electrical lead.

In an embodiment, the method may include mounting a crown portion on the distal end of the electrical lead, the crown portion being of a material having a different stiffness from that of material of a region of deflection of the sleeve, in which region the sleeve bends, and the crown portion defining a plurality of secondary leaves. The method may includes bonding each secondary leaf to an associated petal of the sleeve and forming at least one electrode on an inner surface of each of at least some of the secondary leaves.

According to a third aspect of the invention, there is provided a catheter assembly which includes
a catheter sheath as described above; and
a stylet received in the lumen of the electrical lead.

A distal end of the stylet may carry an electrode. In an embodiment, , when the petals of the catheter sheath are in their closed position, the petals may underlie the button electrode, the stylet being displaceably arranged relative to the catheter sheath.

The electrode may be mounted on the distal end of the stylet via a force absorbing element.

The stylet may be a steering shaft for steering a distal part of the catheter sheath. The steering shaft may define a passage through which conductors for the electrode are able to pass.

In an embodiment, the electrode may be a button electrode. In another embodiment, the electrode may be a needle electrode.

In a further embodiment, a distal end of the electrical lead may carry a distal electrode, the sleeve defining the petals and the petals surrounding the distal electrode when the petals are arranged in their closed configuration.

### Brief Description of the Drawings

Embodiments of the invention are now described by way of example with reference to the accompanying drawings in which :-
Fig. 1 shows a schematic, sectional side view of a distal part of a catheter sheath in accordance with an embodiment of the invention;
Figs 2a-2d show, schematically, various stages of a method, in accordance with another embodiment of the invention, for fabricating a catheter sheath;
Fig. 3 shows a three dimensional view of a distal part of an embodiment of a catheter assembly in a closed configuration;
Fig. 4 shows a side view of the distal part of the catheter assembly of Fig. 3;
Fig. 5 shows a sectional side view of the distal part of the catheter assembly of Fig. 3;
Fig. 6 shows a three dimensional view of the distal part of the catheter assembly of Fig. 3 in an open configuration;
Fig. 7 shows a sectional side view of the catheter assembly of Fig. 3 in an open configuration;
Fig. 8 shows a three dimensional view of a distal part of another embodiment of a catheter assembly in a closed configuration;
Fig. 9 shows a side view of the distal part of the catheter assembly of Fig. 8;
Fig. 10 shows a sectional side view of the distal part of the catheter assembly of Fig. 8;

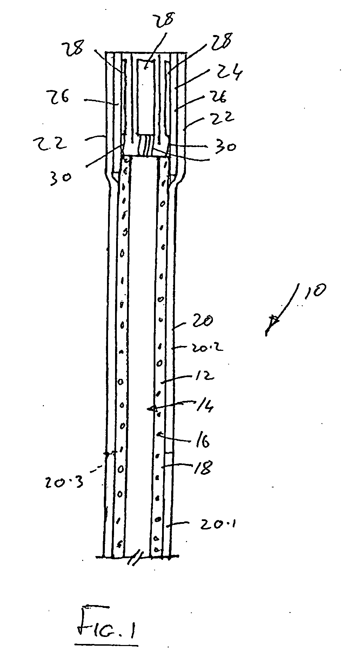

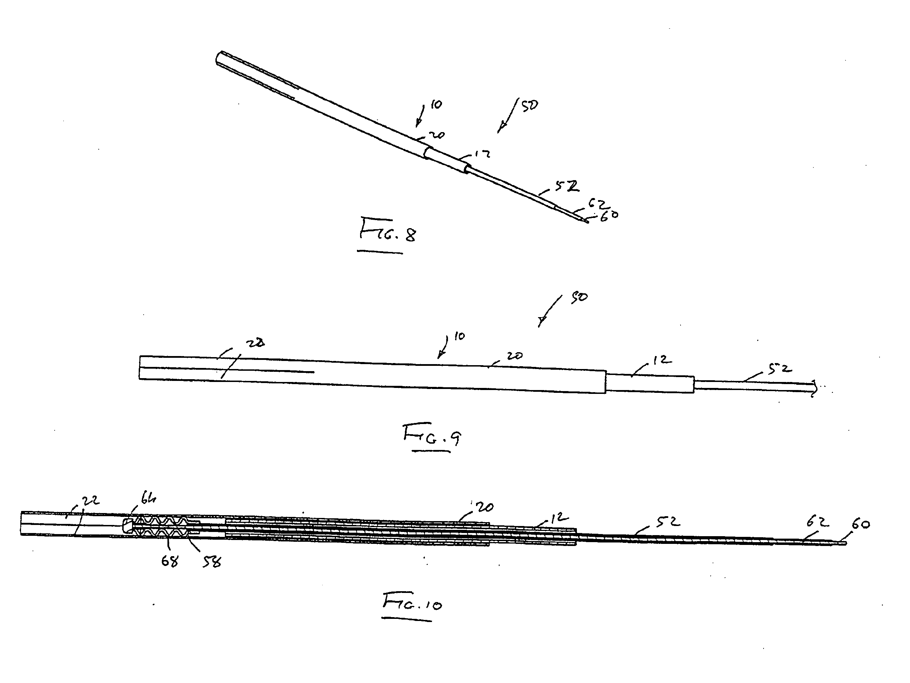

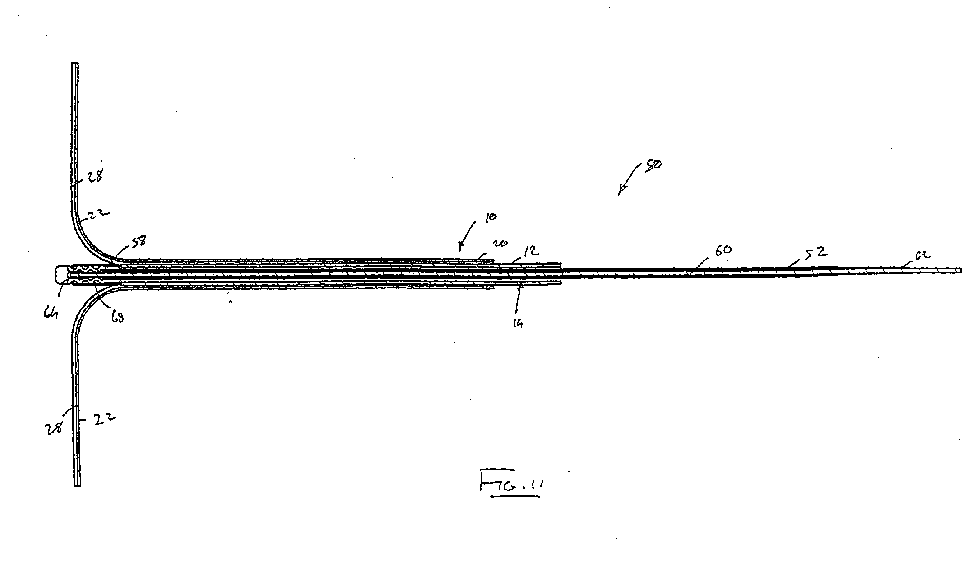

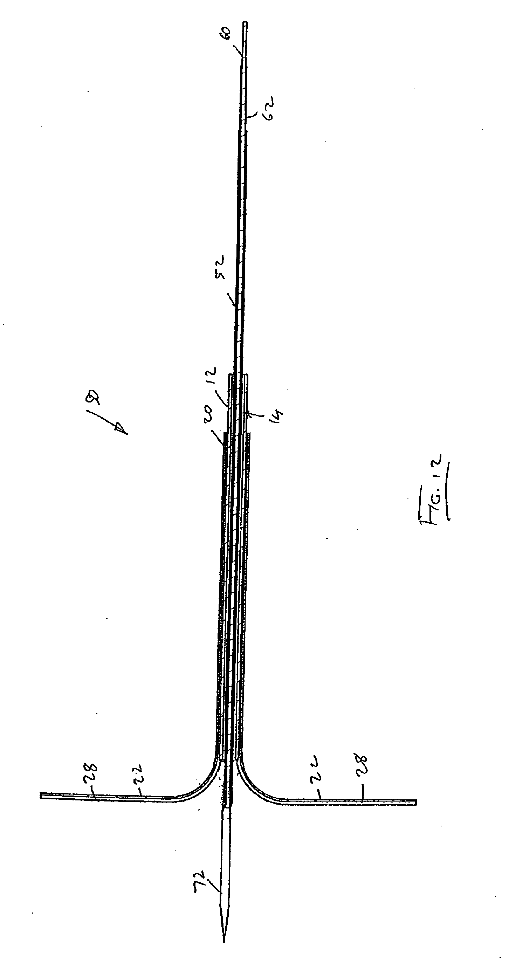

Fig. 1 1 shows a sectional side view of the distal part of the catheter assembly of Fig. 8 in an open configuration; and
Fig. 12 shows a sectional side view of the distal part of yet a further embodiment of a catheter assembly in an open configuration.

### Detailed Description of Exemplary Embodiments

In Fig. 1 of the drawings, reference numeral 10 generally designates a catheter sheath in accordance with an embodiment of the invention. The catheter sheath 10 comprises a tubular electrical lead 12 defining a lumen 14. Electrical conductors 16 are embedded in a wall 18 of the electrical lead 12. Preferably. the electrical lead 12 is manufactured in accordance with the teachings of the Assignee's co-pending International Patent Application No PCT/AU01/01339 dated 19 October 2001 and entitled "An electrical lead". In the technique used in the above International Patent Application, a wall 18 of the electrical lead 12 is formed by an inner tubular member about which the electrical conductors 16 are helically wound with an outer jacket being deposited over the electrical conductors. In so doing, the electrical conductors 16 are embedded in the wall 18 of the electrical lead 12 leaving an unimpeded lumen 14.

The catheter sheath 10 further comprises a sleeve 20 co-axially arranged about the electrode lead 12. The sleeve 20, while being a close fit about the outer surface of the electrode lead 12, is movable with respect to the electrode lead 12, as will be described in greater detail below.

A distal end of the sleeve 20 defines a plurality of petals 22. In the illustrated example, four such petals 22 are defined.

In the embodiment illustrated in Fig.1 of the drawings, a distal end of the electrical lead 18 carries a crown portion 24. A distal end of the crown portion 24 terminates at the distal end of the sleeve 20. The crown portion 24 defines a plurality of secondary leaves 26, there being the same number of leaves 26 as there are petals 22 of the sleeve 20. Further, each leaf 26 lies in register with one of the petals 22 of the sleeve 20.

The sleeve 20 has a proximal part 20.1 (Fig. 1) of a plastics material of a first stiffness, for example, a Shore Hardness of about 63D-72D. At a line of demarcation 20.3, the sleeve 20 has a region of deflection 20.2. The region 20.2 of the sleeve 20 is of a more flexible plastics material having a Shore Hardness of, for example, about 44-53D. The crown portion 24 is of a different hardness of plastics material than the region 20.2 of the sleeve 20. Typically, the crown portion 24 could be of a polyimide plastics material having a Shore Hardness of about 63D-72D. The stiffer proximal part 20.1 of the sleeve 20 facilitates steering of the catheter sheath 10 through a vascular system of a patient's body while the flexible part 20.2 facilitates bending of the sleeve and, accordingly, the catheter sheath 10 at the region of deflection under the action of a stylet.

An inner surface (as defined) of each leaf 26 of the crown portion 24 carries an elongate electrode 28. The electrodes 28 extend in a direction parallel to the longitudinal axis of the catheter sheath 10 when the petals 22 of the sleeve 20, and, accordingly, the leaves 26 of the crown portion 24, are in their closed configuration which is an inoperative configuration. When the petals 22 and leaves 26 are in their closed configuration, they, likewise, extend parallel to the longitudinal axis of the catheter sheath 10. The petals 22 and leaves 26 are moveable to an open, operative configuration in which each petal 22/leaf 26 combination lies transversely to a longitudinal axis of the catheter sheath 10.

Each electrode 28 is connected to its associated distal end of the electrical lead 12 via conductor parts 30 of a group of the conductors 16. As illustrated in Fig. of the drawings, each electrode 28 has four conductor parts 30 associated with it. Two of the conductors 16 are used for providing ablation energy, such as RF energy, to the electrode 28. The remaining two conductors 16 are used for a thermocouple to monitor the temperature at the site where the electrode 28 is operating.

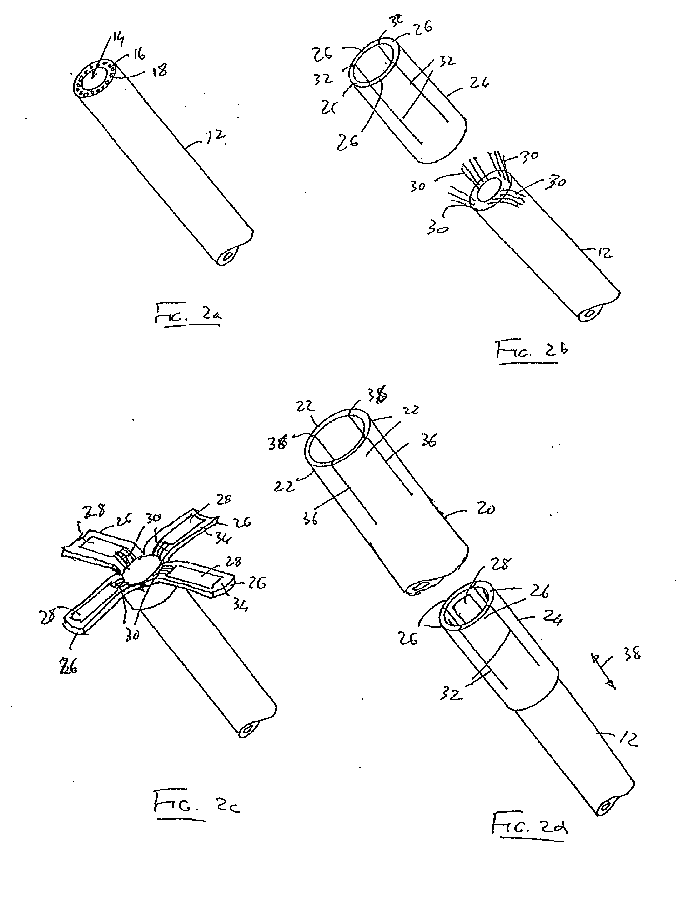

Referring now to Figs 2a-2d of the drawings, a method of fabricating the catheter sheath 10 is described. With reference to Fig. 1 of the drawings, like reference numerals to like parts unless otherwise specified.

In the fabrication of the catheter sheath 10, a length of electrical lead 12 is provided. As described above, the electrical lead 12 has electrical conductors 16 embedded in a wall 18 of the electrical wall 12. While the conductors 16 are shown spaced apart from each other in Fig. 1 of the drawings, that is for illustrative purposes only and it will be appreciated that these conductors 16, being insulated, are helically wound about the inner tube of the electrical tube 12 in a side-by-side manner.

A distal part of the outer plastics jacket, for example, a PEBAX jacket, is removed as is a distal part of the inner tubular member forming the inner part of the wall 18 of the electrical lead 12. This inner part may also be of a PEBAX material. By removing the inner part and the outer jacket, the conductor parts 30 are exposed as illustrated in Fig. 2b of the drawings. The insulation of at least a distal portion of the exposed part 30 of each conductor 16 is removed.

The crown portion 24 is provided and four equally-spaced slits 32 are cut longitudinally in the crown portion 24 to form the leaves 26. For example, the crown portion 24 may be approximately 25mm in length with the slits 32 being 15mm in length.

With these conductor parts 30 arranged in the groups of four, the crown portion 24 is slid over the distal end of the electrical lead 12. A proximal part of the crown portion 24 is adhesively bonded to the distal end of the electrical lead 12. The leaves 26 of the crown portion 24 are moved to an open configuration as shown schematically in Fig. 2c of the drawings.

An electrode 28 is applied to an operatively inner surface 34 of each leaf 26. Each electrode 28 is electrically connected to its associated group of conductor parts 30 via the stripped distal portions of the conductor parts 30. Each electrode 28 is also made mechanically fast with its associated group of conductors 16. The electrical connection of the electrodes 28 to their associated conductors 16 may occur prior to adhering the electrodes 28 to their associated leaves 26 or after having first secured the conductors 16 to the leaves 26. Each electrode 28 may be of a platinum-iridium foil or a platinum coated Nitinol material.

The leaves 26 are moved to their closed configuration as shown in Fig. 2d of the drawings. A sleeve 20 is provided. Longitudinally extending slits 36 are cut in a distal end of the sleeve 20, the slits 26 being spaced 90° apart from each other and defining the petals 22. The length of each slit 36 in the sleeve 20 is approximately the same as the length of each slit 32 in the crown portion 24. The sleeve 20 is slid over the distal end of the electrical lead 12 and its attached crown portion 24. When the sleeve 20 is placed over the crown portion 24, the petals 22 are brought into register with the leaves 26 of the crown portion 24 and outer surfaces of the leaves 26 are secured to inner surfaces of the petals 22.

By relative movement between the electrical lead 12 and the sleeve 20 in the direction of arrows 38 (Fig. 2d), the petal 22/leaf 26 combinations of the catheter sheath 10 can be moved between the open configuration and the closed configuration. More particularly, by urging the electrical lead 12 in a distal direction relative to the sleeve 20, the petals 22/leaves 26 are moved to their open, operative configuration as shown in Fig. 2c of the drawings. Conversely, by moving the electrical lead 12 in a proximal direction relative to the sleeve 20, the petals 22/leaves 26 are moved to their closed, inoperative configuration.

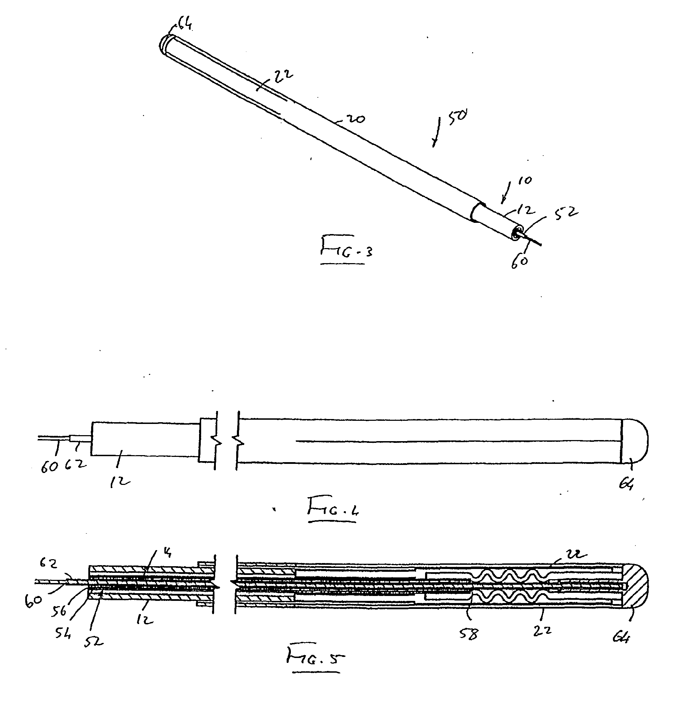

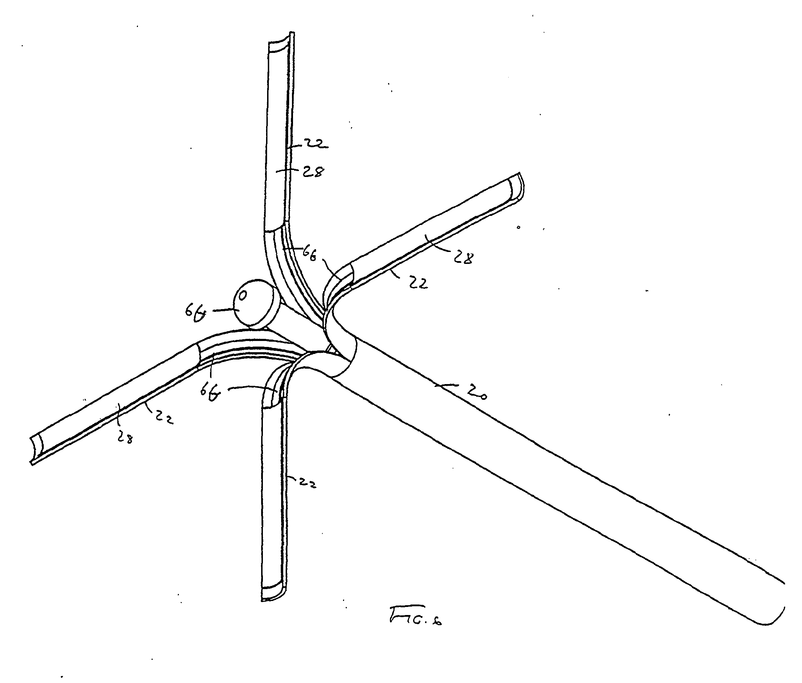

The catheter sheath 10 forms part of a catheter assembly 50, a first embodiment of which is shown in Figs 3-7 of the drawings. The catheter assembly 50 comprises the catheter sheath 10 with a stylet 52 received in the lumen 14 of the electrical lead 12.

In a preferred embodiment, the stylet 52 is a steering shaft. The steering shaft 52 comprises an outer tubular member 54 and an inner, tubular actuator 56 slidably received within the outer tubular member 54. The tubular member 54 and the actuator 56 are fast with each other at a distal end 58 of the steering shaft 52. The tubular member 54 of the steering shaft 52 has a bend-enhancing region (not shown) arranged proximally of the distal end 58. For example, the bend-enhancing region may be a slot cut out of the wall of the tubular member 54, the slot extending in a longitudinal direction and circumscribing about a 270° arc to leave a spine of material. Then, by relative movement between the tubular member 54 and the tubular actuator 56, the distal end 58 can be made to bend in a desired direction at the bend-enhancing region of the steering shaft 52.

The tubular actuator 56 defines a passage or lumen in which a conductor 60 having insulation 62 is received, the insulation 62 insulating the conductor 60 from the steering shaft 52. The conductor 60 passes through the distal end 58 of the steering shaft 52 and mounts a button electrode 64 at its distal end.

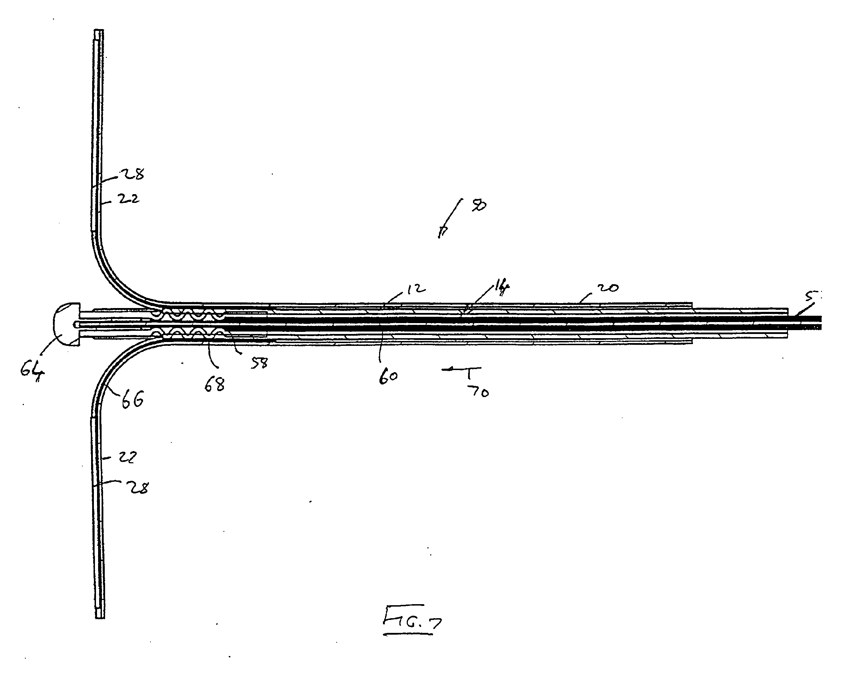

In the embodiment of the catheter assembly 50 shown in Figs 3-7 of the drawings, the button electrode 64 is of a diameter which approximates the outer diameter of the sleeve 20 so that the petals 22 of the sleeve 20 underlie the button electrode 64 when the petals 22 are in their closed configuration. It is to be noted that, in this embodiment of the invention, the crown portion 24 is omitted and the sleeve 22 is mounted directly about the electrical lead 12. Stiffness is provided by using sufficiently stiff electrodes 28, for example, made of a Nitinol material. In addition, as shown most clearly in Fig. 6 of the drawings, each electrode 28 is mounted on a leaf spring 66 which is of a shape memory alloy, for example, a Nitinol material. Each leaf spring 66 is pre-shaped to urge the petals 22 into their closed configuration. It is to be noted that, in Figs. 6 and 7 of the drawings, the conductors 16 are omitted for the sake of clarity.

In the embodiment above, each electrode 28 is formed by metal coating the Nitinol, for example, with platinum and sandwiching the exposed conductors 30 between the electrode 28 and the inner surface of its associated petal 22 with a layer of conductive adhesive. If the Nitinol is omitted, each electrode 28 is formed by covering the exposed conductors 30 with a layer of a conductive adhesive and then coating with a biocompatible material such as platinum.

The electrode 64 is mounted on the distal end 58 of the steering shaft 52 via a force absorbing element in the form of a resiliently flexible bellows-like member 68. The bellows-like member 68 contracts when pressure is exerted on the electrode 64 and, in so doing, inhibits penetration of tissue by the electrode 64, in use.

To effect opening of the petals 22, the electrical lead 12 is urged in the direction of the arrows 70 (Fig. 7) relative to the sleeve 20 by about 4mm. This serves to open the petals 22 into a plane lying substantially at right angles to the longitudinal axis of the catheter assembly 50. However, the button electrode 64 lies out of the plane. To get the button electrode 64 more or less in the same plane as the electrodes 28 when they are in their open configuration, the steering shaft 52 is moved in the direction opposite to that shown by arrow 70 by an amount of approximately 7-11 mm.

In Figs. 8-11 of the drawings, a further embodiment of a catheter assembly 50 is illustrated. With reference to Figs. 5-7 of the drawings, like reference numerals refer to like parts, unless otherwise specified. In this embodiment, when the petals 22 are in their closed configuration, they surround the button electrode 64 as shown most clearly in Fig. 10 of the drawings. In this embodiment, the relative movement only occurs between the sleeve 20 and the electrical lead 12. It is not necessary for there to be relative longitudinal movement between the cathode sheath 10 and the steering shaft 52 because, as illustrated more clearly in Fig. 11 of the drawings, when the petals 22 of the sleeve 20 move to their open configuration, the electrodes 28 carried by the petals 22 lie more or less in plane with the tip of the button electrode 64.

Both the embodiments shown in Figs. 3-7 of the drawings and Figs. 8-11 of the drawings relate to catheters used for ablation treatment such as, for example, in the treatment of heart arrhythmias. In this embodiment, the electrodes 28 and 64 are used to effect the ablation treatment at the site of the patient's body. At any one time, two electrodes are used such as one of the electrodes 28 and the electrode 64 or two of the electrodes 28 adjacent to each other or opposed to each other, as the case may be. The electrodes 28, 64 are energised with out-of-phase energy as described in greater detail in the Assignee's co-pending International Patent Application No PCT/AU03/01421 dated 28 October 2003 and entitled "System for, and method of, heating a biological site in a patient's body."

As described in that International Application, the energy supplied to any one electrode is 180° out of phase with the energy supplied to any other electrode. The sum of the energy applied is, however, still the same and, using this method, longer but shallower lesions are formed resulting in less trauma at the treated site in the patient's body.

Fig. 12 shows yet a further embodiment of a catheter assembly 50. Once again, with reference to the previous drawings, like reference numerals to like parts, unless otherwise specified. In this embodiment of the invention, the catheter assembly 50 is intended for use in the treatment of ventricular tachycardia. To enable tissue to be treated, instead of the button electrode 64, a needle electrode 72 is carried on the distal end of the steering shaft 52. Because penetration of the tissue is required, the bellows-like member 68 is omitted.

With this arrangement, the needle electrode 72 enters the tissue to be treated and is used in combination with one or more of the electrodes 28.

It is an advantage of the invention that a catheter sheath 10 is provided which is collapsible to be inserted through the vasculature of a patient's body and to have a distal end which opens out into a flower-like arrangement at the desired site to enable a wide area of heat treatment to be effected at the site. This is achieved with minimum manipulation of the distal end of the catheter sheath when it is at the desired site making it easier for a clinician to use and to position.

In addition, relatively long electrodes 28 are provided for enabling long, shallow lesions to be formed at the site resulting in less trauma but more effective treatment of heart arrhythmias to be performed.

It is still a further advantage of the invention that the resilience of the petals 22 facilitates good tissue/electrode contact. This may be enhanced by use of the leaf springs 66 associated with each electrode 28 or, instead, the petals 22 and/or leaves 26 may be of sufficient resilience to promote tissue/electrode contact and to move the petals/leaves to their closed configurations when the distal ends of the catheter assembly is moved out of contact with the tissue at the site at the patient's body. Such resilience also enhances tissue/electrode contact and helps overcome the effect of tissue irregularities at the site.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. A catheter sheath which includes
an electrical lead carrying a plurality of electrical conductors; and
a sleeve co-axially arranged about the electrical lead, at least one of a distal end of the lead and a distal end of the sleeve defining a plurality of displaceable petals, the petals being displaceable between a first, inoperative position in which the petals extend parallel to a longitudinal axis of the sleeve and a second, operative position in which the petals lie transversely to the longitudinal axis of the sleeve; and
at least one of the petals carrying at least one electrode, the, or each, electrode being in electrical connection with a group of the conductors of the lead.

2. The catheter sheath of claim 1 in which the electrical lead is tubular defining a lumen and the electrical conductors are embedded in a wall of the electrical lead.

3. The catheter sheath of claim 1 or claim 2 in which the distal end of the sleeve extends beyond the distal end of the lead, the distal end of the sleeve defining the petals.

4. The catheter sheath of claim 3 in which the sleeve is of a plastics material of a first stiffness along its length and a more flexible material in a region of deflection in which region the sleeve is able to bend.

5. The catheter sheath of claim 4 which includes a crown portion of a plastics material of a different stiffness to the flexible material of the region of deflection, the crown portion being attached to a distal end of the electrical lead and projecting distally of the distal end of the electrical lead, the crown portion defining a plurality of secondary leaves, each secondary leaf being in register with one of the petals of the sleeve.

6. The catheter sheath of claim 5 in which the crown portion is arranged within the sleeve and the electrodes are carried on inner surfaces of the secondary leaves of the crown portion, each secondary leaf being fast with its associated petal of the sleeve.

7. The catheter sheath of claim 3 in which the sleeve is of a plastics material of a first stiffness along its length, a more flexible material in a region of deflection in which region the sleeve bends and in which the sheath includes a crown portion of a plastics material of a different stiffness to the flexible material of the region of deflection attached to a distal end of the region of deflection, the crown portion defining the petals the sleeve.

8. A method of fabricating a catheter sheath, the method including
providing an electrical lead carrying electrical conductors;
providing a sleeve and arranging the sleeve co-axially with respect to the electrical lead, at least one of the sleeve and the lead defining a plurality of discrete petals at its distal end;
forming at least one electrode on at least one of the petals; and
electrically connecting the, or each, electrode to a group of the conductors.

9. The method of claim 8 in which the electrical lead is tubular defining a lumen with the conductors being embedded in a wall of the electrical lead, the method including removing material of a distal part of the wall of the electrical lead to expose distal regions of the conductors which form free parts of the conductors.

10. The method of claim 9 which includes arranging the parts of the conductors to extend distally from the distal end of the electrical lead and, after exposing the parts of the conductors, stripping insulation from the exposed parts of the conductors.

11. The method of any one of claims 8 to 10 which includes arranging the sleeve about the electrical lead.

12. The method of any one of claims 8 to 11 which includes mounting a crown portion on the distal end of the electrical lead, the crown portion being of a material having a different stiffness from that of material of a region of deflection of the sleeve, in which region the sleeve bends, and the crown portion defining a plurality of secondary leaves.

13. The method of claim 12 which includes bonding each secondary leaf to an associated petal of the sleeve and forming at least one electrode on an inner surface of each of at least some of the secondary leaves.

14. A catheter assembly which includes
a catheter sheath as claimed in any one of claims 1 to 7; and
a stylet received in the lumen of the electrical lead.

15. The assembly of claim 14 in which a distal end of the stylet carries an electrode.

16. The assembly of claim 15 in which, when the petals of the catheter sheath are in their closed position, the petals underlie the button electrode, the stylet being displaceably arranged relative to the catheter sheath.

17. The assembly of claim 15 or claim 16 in which the electrode is mounted on the distal end of the stylet via a force absorbing element.

18. The assembly of any one of claims 15 to 17 in which the stylet is a steering shaft for steering a distal part of the catheter sheath, the steering shaft defining a passage through which conductors for the electrode are able to pass.

19. The assembly of any one of claims 15 to 18 in which the electrode is a button electrode.

20. The assembly of any one of claims 15 to 18 in which the electrode is a needle electrode.

21. The assembly of claim 14 in which a distal end of the electrical lead carries a distal electrode, the sleeve defining the petals and the petals surrounding the distal electrode when the petals are arranged in their closed configuration.
